(19) Europäisches Patentamt
European Patent Office
Office européen des brevets

(11) Veröffentlichungsnummer: **0 277 317 B1**

# (12) EUROPÄISCHE PATENTSCHRIFT

(45) Veröffentlichungstag der Patentschrift: **03.04.91**

(51) Int. Cl.$^5$: **C07D 233/52**, C07D 239/16, C07D 233/48, C07D 239/18, C07D 401/04, C07D 401/06, C07D 401/12, C07D 403/04, C07D 403/06, C07D 403/12, C07D 405/06

(21) Anmeldenummer: **87118054.3**

(22) Anmeldetag: **07.12.87**

## (54) Nitro-Derivate von 2-Iminoimidazolidinen und 2-Iminotetrahydropyrimidinen.

(30) Priorität: **19.12.86 JP 301333/86**

(43) Veröffentlichungstag der Anmeldung: **10.08.88 Patentblatt 88/32**

(45) Bekanntmachung des Hinweises auf die Patenterteilung: **03.04.91 Patentblatt 91/14**

(84) Benannte Vertragsstaaten: **BE CH DE FR GB IT LI NL**

(56) Entgegenhaltungen:
**EP-A- 0 235 725**
**DE-A- 2 205 745**

**CHEMICAL & PHARMACEUTICAL BULLETIN, Band 27, Nr. 4, 1. April 1979, Seiten 841-847, Pharmaceutical Society of Japan, Tokyo, JP; A. KOSASAYAMA et al.: "Cyclic guanidines. IV. Synthesis of hypoglycemic N-benzhydryl bicyclic guanidines"**

(73) Patentinhaber: **NIHON TOKUSHU NOYAKU SEIZO K.K.**
**Itohpia Nihonbashi Honcho Building 7-1, Nihonbashi Honcho 2-chome**
**Chuo-ku Tokyo 103(JP)**

(72) Erfinder: **Shiokawa, Kozo**
**210-6, Shukugawara**
**Tama-ku Kawasaki-shi Kanagawa-ken(JP)**
Erfinder: **Tsuboi, Shinichi**
**3-26-1, Hirayama**
**Hino-shi Tokyo(JP)**
Erfinder: **Moriya, Koichi**
**5-7-11, Ueno**
**Taito-ku Tokyo(JP)**
Erfinder: **Shibuya, Katsuhiko**
**39-15, Namiki-cho**
**Hachioji-shi Tokyo(JP)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 277 317 B1

CHEMICAL & PHARMACEUTICAL BULLETIN, Band 26, Nr. 12, 1. Dezember 1978, Seiten 3658-3665, Pharmaceutical Society of Japan, Tokyo, JP; F. ISHIKAWA et al.: "Cyclic guanidines. I. Synthesis of hypoglycemic 1-substituted 2-imino-1,3-diazacycloalkanes"

CHEMICAL & PHARMACEUTICAL BULLETIN, Band 26, Nr. 12, 1. Dezember 1978, Seiten 3666-3674, Pharmaceutical Society of Japan, Tokyo, JP; F. ISHIKAWA et al.: "Cyclic guanidines. II. Synthesis of hypoglycemic acyl or alkyl derivatives of 1-substituted 2-imino-1,3-diazacycloalkane"

(74) Vertreter: Ernst, Hilmar, Dr. et al
Bayer AG Konzernverwaltung RP Patentabteilung
W-5090 Leverkusen 1, Bayerwerk(DE)

## Beschreibung

Die vorliegende Erfindung betrifft neue 2-Nitroiminoimidazolin-Derivate und Verfahren zu ihrer Herstellung.

Es ist bereits bekannt, daß bestimmte Cyanoimino-Verbindungen als Zwischenprodukte für aktive Verbindungen wie Insektizide, antidiabetische Mittel, virusunterdrückende Mittel und diuretische Mittel von Nutzen sind (siehe die JP-OS 91064/1983).

Aus den Publikationen DE-A 2 205 745, Chemical and Pharmaceutical Bulletin, Band 26, Nr. 12, 01/12/78, S. 3663, Chemical and Pharmaceutical Bulletin, Band 27, Nr. 4, 01/04/79, S. 842 und EP-A 0 235 725 sind ebenfalls Cyaniminoverbindungen bekannt, die eine gewisse Strukturverwandtschaft mit den erfindungsgemäßen Stoffen aufweisen.

Weiterhin sind bestimmte Nitroimino-Verbindungen in Can.J.Chem., Band 39, S. 1787-1796, offenbart.

Nunmehr wurden neue 2-Nitroiminoimidazolin-Derivate der allgemeinen Formel (I)

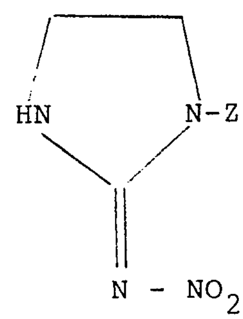

gefunden, in welcher Z für einen der folgenden Substituenten steht:

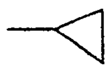 , $-CH_2-CH_2Cl$, 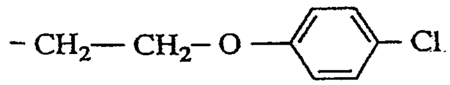 ,

$-CH_2-CH_2-CH_2-SCH_3$, $-CH_2-CH_2-CN$, 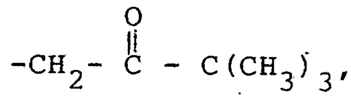

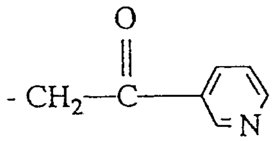 , 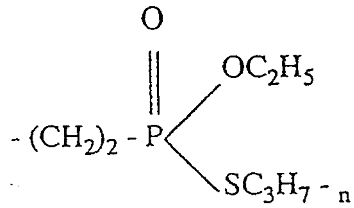 , $-CH_2-Si(CH_3)_3$,

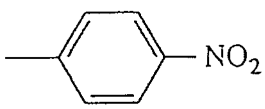 , 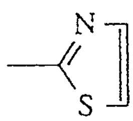 , 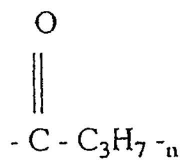 , 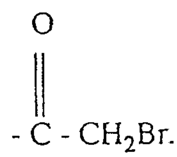

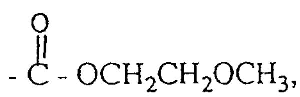

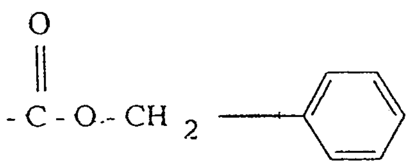 , 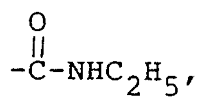 , 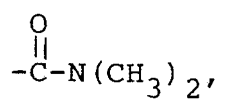 , $-SO_2CH_3$

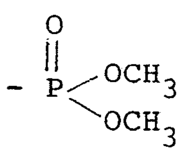 , 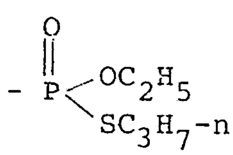 , 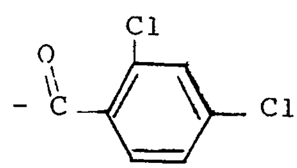 ,

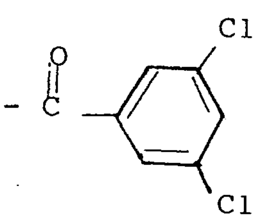 , 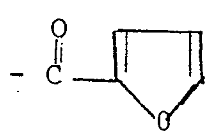 .

Die Verbindungen der Formel (I) werden mittels eines Verfahrens erhalten, bei dem die Verbindung der Formel (II)

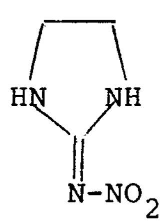

(II)

mit Verbindungen der Formel (III)

$$Z - M^1 \qquad \text{(III)},$$

in der Z die im Vorstehenden angegebenen Bedeutungen hat und $M_1$ für Halogen, vorzugsweise Chlor oder Brom steht,
in Gegenwart eines inerten Lösungsmittels und gegebenenfalls in Gegenwart einer Base umgesetzt werden oder
b) Verbindungen der Formel (IV)

$$H_2N-CH_2-CH_2-NH-Z \qquad \text{(IV)},$$

in der Z die im Vorstehenden angegebenen Bedeutungen hat, mit Verbindungen der Formel (V)

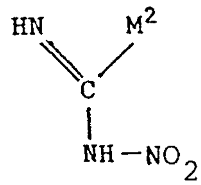

(V)

in der $M^2$ für Amino, Alkylthio oder Benzylthio steht, in Gegenwart eines inerten Lösungsmittels umgesetzt werden.

Die neuen Nitroimino-Verbindungen sind sehr wertvoll als Zwischenprodukte für bestimmte insektizide Nitroimino-Verbindungen, wie im einzelnen weiter unten dargelegt wird.

Wenn beispielsweise in dem Verfahren a) 2-Nitroimino-imidazolidin und 1-Chloro-3,3-dimethyl-2-butanon als Ausgangsstoffe eingesetzt werden, kann der Reaktionsverlauf durch die folgende Gleichung dargestellt werden:

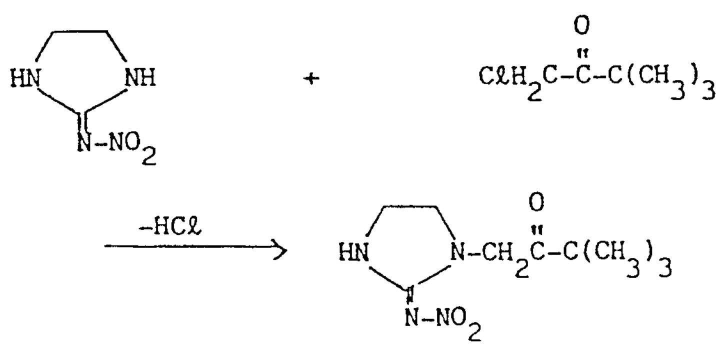


Wenn beispielsweise in dem Verfahren b) N-2-Chlorethyl diamin und N-Nitro-S-methylisothioharnstoff als Ausgangsstoffe eingesetzt werden, kann der Reaktionsverlauf durch die folgende Gleichung dargestellt werden:

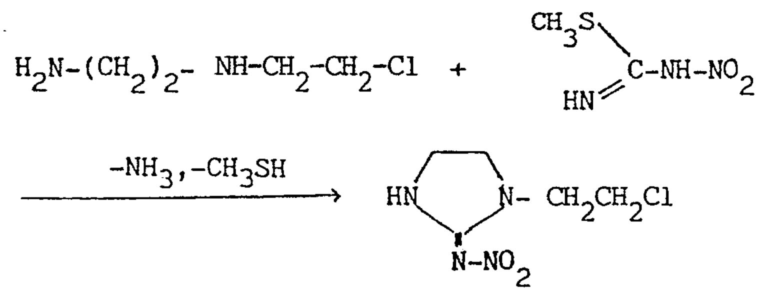


In dem Verfahren a) bezeichnen die Verbindungen der Formel (III) als Ausgangsstoffe die Verbindungen auf der Grundlage der oben angegebenen Definitionen von Z und $M^1$.

In der Formel (III) haben z und $M^1$ vorzugsweise die oben angegebenen bevorzugten Bedeutungen.

Die Verbindungen der Formel (III) sind auf dem Gebiet der organischen Chemie wohlbekannt.

In dem Verfahren b) bezeichnen die Verbindungen der Formel (IV) als Ausgangsstoffe die Verbindungen auf der Grundlage der oben angegebenen Definitionen von Z.

In der Formel (IV) hat Z vorzugsweise die oben angegebenen bevorzugten Bedeutungen.

Die Verbindungen der Formel (IV) umfassen bekannte Verbindungen und werden im allgemeinen in einfacher Weise enthalten, wenn l,2-Diaminoethan (VII)

$$H_2N - CH_2 - CH_2 - NH_2 \qquad (VII),$$

mit den Verbindungen der oben angegebenen Formel (III) in Gegenwart eines inerten Lösungsmittels und gegebenen-falls in Gegenwart einer Base umgesetzt wird.

In dem Verfahren b) sind die ebenfalls als Ausgangsstoffe eingesetzten Verbindungen der Formel (V) bekannt (siehe beispielsweise J. Am. Chem. Soc., Band 76, S. 1877).

Das Verfahren a) gemaß der Erfindung kann unter Verwendung eines geeigneten Lösungsmittels oder Verdünnungsmittels durchgeführt werden. Praktisch alle inerten organischen lösungsmittel können als Lösungsmittel oder Verdünnungsmittel verwendet werden. Beispiele für Lösungsmittel oder Verdünnungsmittel sind Wasser; aliphatische, cycloaliphatische und aromatische, gegebenenfalls chlorierte, Kohlenwasserstoffe, etwa Hexan, Cyclohexan, Petrolether, Ligroin, Benzol, Toluol, Xylol, Methylenchlorid, Chloroform, Kohlenstofftetrachlorid, Ethylenchlorid, Trichloroethylen, Chlorbenzol und dergleichen; Ether, etwa Diethylether, Methylethylether, Diisopropylether, Dibutylether, Propylenoxid, Dioxan, Tetrahydrofuran und dergleichen; Nitrile, etwa Acetonitril, Propionitril, Acrylnitril und dergleichen; Alkohole, etwa Methanol, Ethanol, Isopropanol, Butanol, Ethylenglycol und dergleichen; Säureamide, etwa Dimethylformamid, Dimethylacetamid und dergleichen; und

Sulfone und Sulfoxide, etwa Dimethylsulfoxid, Sulfolan und dergleichen.

Das Verfahren a) gemäß der Erfindung kann in Gegenwart einer Base wie Alkalimetallhydroxiden oder -carbonaten durchgeführt werden, beispielsweise Natriumhydroxid, Kaliumhydroxid oder dergleichen.

Die Reaktionstemperatur kann innerhalb eines breiten Bereichs variiert werden. Im allgemeinen wird die Reaktion bei einer Temperatur von etwa O °C bis 100 °C, vorzugsweise von etwa 10°C bis 80°C, durchgeführt.

Im allgemeinen läßt man die Reaktion unter normalem Druck ablaufen, obwohl es auch möglich ist, einen höheren oder niedrigeren Druck anzuwenden.

Wenn das Verfahren a) zur Herstellung der Verbindungen der Formel (I) in einem inerten Lösungsmittel durchgeführt wird, ist es möglich, etwa 0,9 bis 1,1 mol der Verbindungen (II) pro 1 mol der Verbindungen (III) in Gegenwart einer Base einzusetzen.

Bei der Durchführung des Verfahrens b) umfassen geeignete Lösungsmittel die gleichen Lösungsmittel, wie sie oben für das Verfahren a) aufgeführt sind.

Das Verfahren b) kann allgemein bei einer Temperatur von etwa 0 °C bis 120 °C, vorzugsweise von etwa 30 °C bis 100 °C, durchgeführt werden. Es ist vorteilhaft, das Verfahren b) unter normalem Druck durchzuführen, obwohl es auch möglich ist, dieses Verfahren unter höherem oder niedrigerem Druck durchzuführen.

Wenn das Verfahren b) zur Gewinnung der Verbindungen der Formel (I) durchgeführt wird, werden im

allgemeinen etwa 1 bis 1,2 mol, vorzugsweise etwa 1 bis 1,1 mol, der Verbindungen (V) pro 1 mol der Verbindungen (IV) eingesetzt. In dem Verfahren b) können die Verbindungen der Formel (IV) und die Verbindungen (V) beispielsweise in Wasser miteinander erhitzt werden, um sie miteinander umzusetzen.

Die Verbindungen der nachstehenden Formel (E), die unter Verwendung der Verbindungen der Formel (I) als Zwischenprodukte hergestellt werden können, zeigen starke insektizide Wirkung:

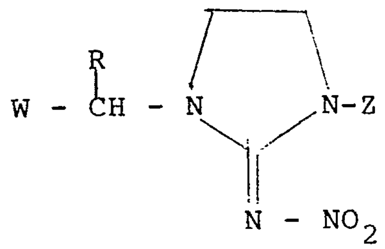

(E)

In der vorstehenden Formel (E) hat Z die im Vorstehenden angegebenen Bedeutungen, R bezeichnet Wasserstoff oder Alkyl und W steht für einen gegebenenfalls substituierten 5- oder 6-gliedrigen heterocyclischen Rest mit wenigstens einem, aus der aus N, 0 und S bestehenden Klasse ausgewählten Hetero-Atom.

Die Herstellung von Produkten der Formel (E) kann wie folgt formuliert werden:

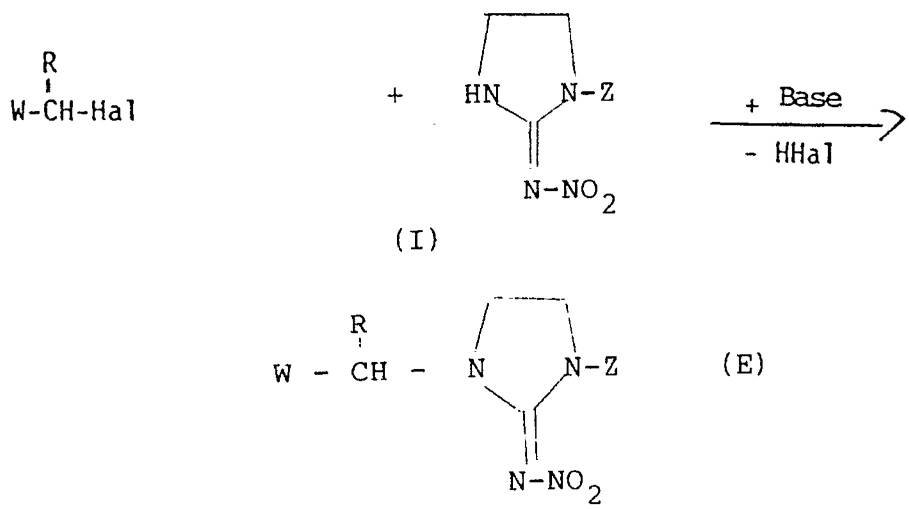


(I)

(E)

In den obigen Formeln haben Z, W und R die im Vorstehenden angegebenen Bedeutungen, und Hal steht für ein Halogen-Atom.

Die Verbindungen der vorstehenden Formel (E) können beispielsweise entsprechend den weiter unten aufgeführten Bezugsbeispielen 1 und 2 erhalten werden.

Weiterhin zeigen auch die Verbindungen der Formel (I) an sich insektizide Wirkung.

Die Erfindung wird durch die folgenden Beispiele näher erläutert, ist jedoch nicht auf diese beschränkt.

## Herstellungsbeispiele

### Beispiel 1

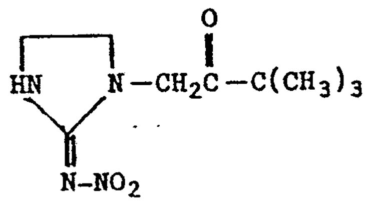

Eine Mischung aus 2,6 g 2-Nitroiminoimidazolidin, 3,0 g wasserfreiem Kaliumcarbonat und 30 ml trockenem Acetonitril wurde bei Raumtemperatur 30 min gerührt. Dann wurden 2,7 g I-Chloro-3,3-dimethyl-2-butanon unter Rühren bei Raumtemperatur zu der Mischung hinzugefügt. Die Reaktionsmischung wurde erhitzt und 1 h unter Rückfluß gehalten. Das Acetonitril wurde unter vermindertem Druck abdestilliert, und der Rückstand wurde mit Wasser vermischt. Das gewünschte Produkt wurde durch Filtration abgetrennt. Das kristalline Produkt wurde mit Ether gewaschen und getrocknet. 3,5 g I-(3,3-Di-methyl-2-butanon-I-yl)-2-nitroiminoimidazolidin wurden erhalten. Schmp. 158-159 °C.

Weiterhin können verschiedenartige Verbindungen der Formel (I)

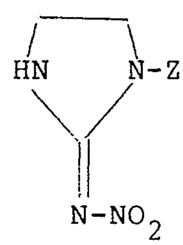

(I)

nach den gleichen Methoden, wie sie z.B. im Beispiel 1 aufgezeigt wurden, hergestellt werden.

Tabelle 1

| Z | Physikalische Konstante |
|---|---|
| Cyclopropyl | |
| 2-Chloroethyl | Schmp. 138-140 °C |
| 2-(4-Chlorophenoxy)ethyl | Schmp. 95-96 °C |
| 3-Methylthiopropyl | Schmp. 93-95 °C |
| 2-Cyanoethyl | Schmp. 135-139 °C |
| 3,3-Dimethyl-2-butanon-1-yl | Schmp. 158-159 °C |
| 3-Pyridylcarbonylmethyl | Schmp. 176-177 °C |
| 2-(O-Ethyl-S-propylthio-phosphonooxy)ethyl | |
| Trimethylsilylmethyl | |
| 4-Nitrophenyl | |
| 2-Thiazolyl | Schmp. 159-162 °C |
| n-Propylcarbonyl | Schmp. 126-130 °C |
| Bromoacetyl | Schmp. 136-140 °C (Zers.) |
| 2-Methoxyethoxycarbonyl | |
| Benzyloxycarbonyl | Schmp. 179-182 °C |
| Ethylaminocarbonyl | Schmp. 145-148 °C |
| Dimethylaminocarbonyl | Schmp. 147-148 °C |
| Methylsulfonyl | |
| O,O-Dimethylphosphono | |
| O-Ethyl-S-propylthio-phosphono | |
| 2,4-Dichlorobenzoyl | Schmp. 184-186 °C |
| 3,5-Dichlorobenzoyl | |
| 2-Furylcarbonyl | Schmp. 160-162 °C |

Bezugsbeispiel

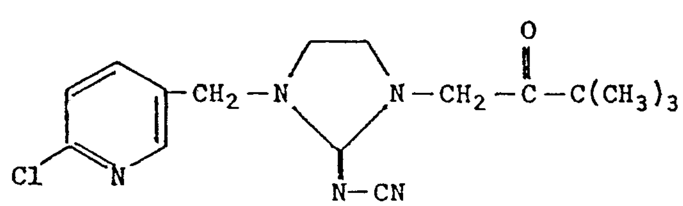

Eine Mischung aus 2,1 g 1-(3,3-Dimethyl-2-butanon-1-yl)-2-cyanoiminoimidazolidin, 1,6 g 2-Chloro-5-chloromethylpyridin, 1,4 g wasserfreies Kaliumcarbonat und 30 ml Acetonitril werden 8 h unter Rühren zum

Rückfluß erhitzt. Nach Beendigung der Reaktion wurde das Acetonitril unter vermindertem Druck abdestilliert. Der Rückstand wurde mit Dichloromethan vermischt und mit Wasser gewaschen. Nach dem Konzentrieren durch Entfernen des Dichloromethans wurde die Reaktionsmischung unter Einsatz einer Silicagel-Säule aufgearbeitet (mit Chloroform:Ethanol = 9:1), so daß 0,9 g des gewünschten Produkts als farbloses viskoses Material erhalten wurden; $n_D^{20}$ = 1,5446.

Nach den gleichen Verfahren wie im Bezugsbeispiel können verschiedenartige Verbindungen der Formel

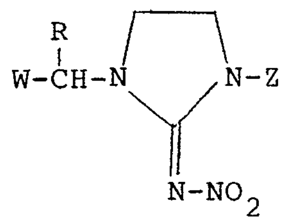

(E)

erhalten werden, wie sie in Tabelle 2 aufgeführt sind.

Tabelle 2

| W | R | Z | Physikalische Konstante |
|---|---|---|---|
| 2-Chloro-5-pyridyl | H | 2-Chloroethyl | |
| 2-Chloro-5-pyridyl | H | 3-Methylthio-propyl | $n_D^{20}$-1,6067 |
| 2-Chloro-5-pyridyl | H | 3,3-Dimethyl-2-butanon-1-yl | Schmp. 105-110 °C |
| 2-Chloro-5-pyridyl | H | 2-Fluoryl | Schmp. 162-163 °C |
| 2-Chloro-5-pyridyl | H | Methylsulfonyl | Schmp. 191-192 °C |
| 3-Methyl-5-isoxazolyl | H | Methylsulfonyl | Schmp. 156-158 |
| 2-Chloro-5-pyridyl | H | 0-Ethyl-S-propyl-thiophosphono | $n_D^{20}$= 1,5615 |
| 2-Chloro-5-thiazolyl | H | 2-Cyanoethyl | $n_D^{20}$= 1,6176 |

Beispiel 2 (Biologischer Test)

Test mit organophosphat-resistenten grünen Reis-Blatt wanzen (Nephotettix cincticeps):

**Herstellung einer Test-Chemikalie:**

| | | |
|---|---|---|
| Lösungsmittel: | Xylol | 3 Gew.-Teile |
| Emulgator: | Polyoxyethylen-alkylphenylether | 1 Gew.-Teil |

Zur Herstellung eines geeigneten Präparats einer aktiven Verbindung wurde 1 Gew.-Teil der aktiven Verbindung mit der angegebenen Menge Lösungsmittel und der angegebenen Menge Emulgator vermischt, und die Mischung wurde mit Wasser auf eine vorher festgesetzte Konzentration verdünnt.

Test-Verfahren :

Eine Wasser-Verdünnung jeder der aktiven Verbindungen mit einer vorher festgelegten Konzentration, die wie oben beschrieben hergestellt worden war, wurde auf Reispflanzen von etwa 10 cm Höhe, die jeweils in Töpfen von 12 cm Durchmesser gezogen worden waren, in einer Menge von 10 ml pro Topf gesprüht. Die aufgesprühte Chemikalie wurde trocknen gelassen, ein Drahtkorb von 7 cm Durchmesser und 14 cm Höhe wurde über jeden der Töpfe gestülpt, und 30 ausgewachsene weibliche Exemplare der Reis-Kleinzikade (Nephotettix cincticeps) eines Stammes, der gegen Organophosphat-Chemikalien Resistenz zeigte, wurden unter dem Drahtkorb ausgesetzt. Die Töpfe wurden in einem Raum mit konstanter Temperatur aufbewahrt. Zwei Tage später wurde die Zahl der toten Insekten bestimmt, und das Tötungsverhältnis wurde berechnet.

Beispiel 3 (Biologischer Test)

Test mit organophosphat-resistenten Weißrücken-Laternenträgern (Sogatella furcifera):

Test-Verfahren :

Eine Wasser-Verdünnung jeder der aktiven Verbindungen mit einer vorher festgelegten Konzentration, die wie in dem vorhergehenden Beispiel beschrieben hergestellt worden war, wurde auf Reispflanzen von etwa 10 cm Höhe, die jeweils in Töpfen von 12 cm Durchmesser gezogen worden waren, in einer Menge von 10 ml pro Topf gesprüht. Die aufgesprühte Chemikalie wurde trocknen gelassen, über jeden der Töpfe wurde ein Drahtkorb von 7 cm Durchmesser und 14 cm Höhe gestülpt, und 30 ausgewachsene weibliche Exemplare des Weißrücken-Laternenträgers (Sogatella furcifera) eines Stammes, der gegen Organophosphat-Chemikalien Resistenz zeigte, wurden unter dem Korb ausgesetzt, und die Töpfe wurden in einen Raum mit konstanter Temperatur gestellt. Zwei Tage später wurde die Zahl der toten Insekten bestimmt, und das Tötungsverhältnis wurde berechnet.

Ergebnisse:

In den oben aufgeführten biologischen Tests (Beispiele 2 und 3) wurde gefunden, daß die in Tabelle 2 aufgeführten aktiven Verbindungen eine ausgezeichnete insektizide Wirkung bei einer Konzentration von 200 ppm und eine gute insektizide Wirkung sogar noch bei einer Konzentration von weniger als 200 ppm zeigen.

## Ansprüche

1. 2-Nitroiminoimidazolidin-Derivate der Formel

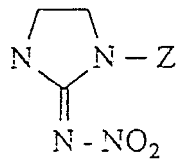

in welcher Z für einen der folgenden Substituenten steht:

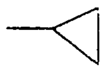 , $-CH_2-CH_2Cl$, 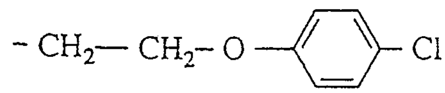

$-CH_2-CH_2-CH_2-SCH_3$, $-CH_2-CH_2-CN$, 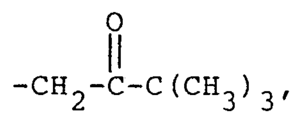,

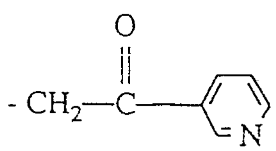 , 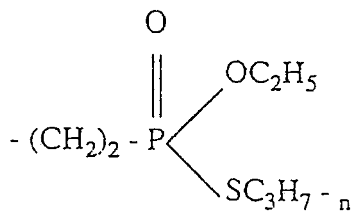 , $-CH_2-Si(CH_3)_3$,

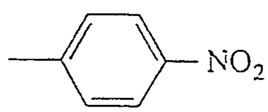 , 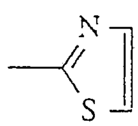 , 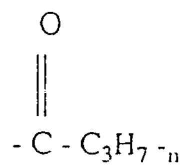 , 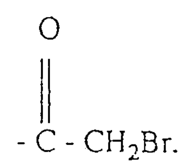 .

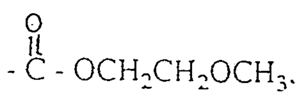,

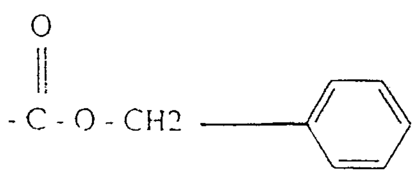 .

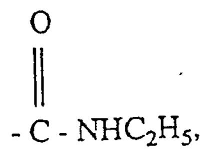, 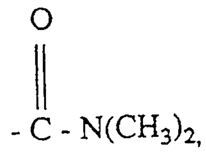, $-SO_2CH_3$,

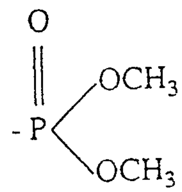, 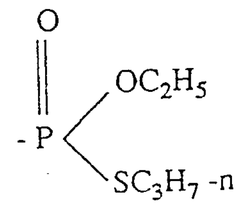.

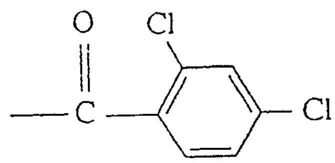,

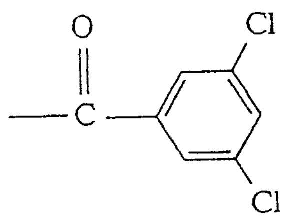,

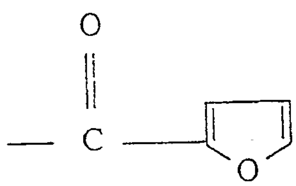.

## Claims

**1.** 2-Nitroiminoimidazolidine derivatives of the formula

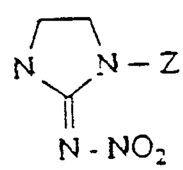

in which Z represents one of the following substituents:

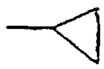 , $-CH_2-CH_2Cl$. 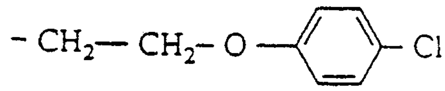

$-CH_2-CH_2-CH_2-SCH_3$, $-CH_2-CH_2-CN$. 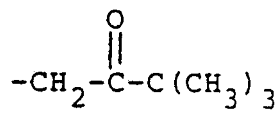,

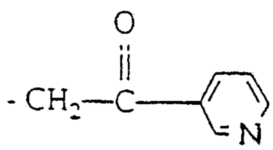 . 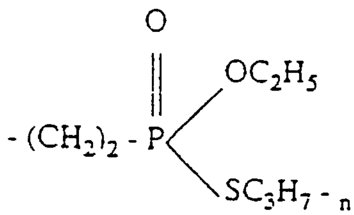 . $-CH_2-Si(CH_3)_3$.

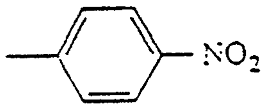 , 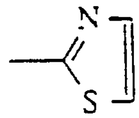 , 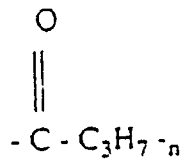 , 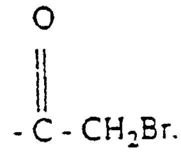 .

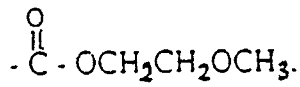 .

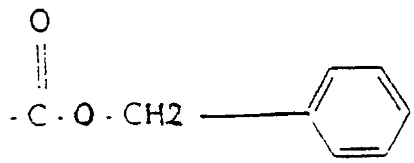 .

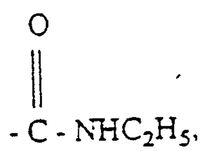 , 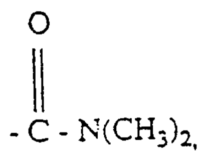 , $-SO_2CH_3$ ,

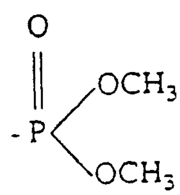 , 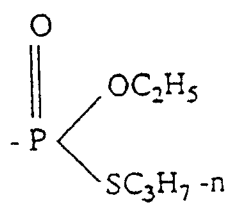 .

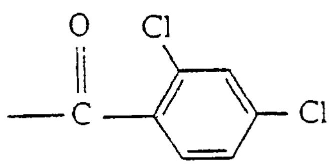 ,

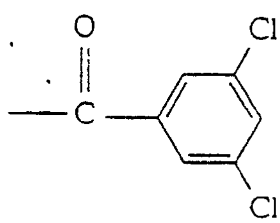 ,

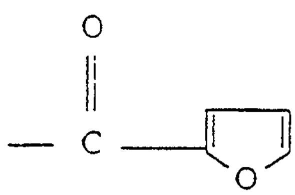 .

## Revendications

**1.** Dérivés de 2-nitroiminoimidazoline de formule

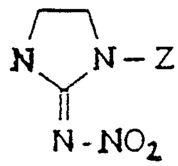

dans laquelle Z représente l'un des substituants suivants:

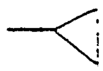 . $-CH_2-CH_2Cl$, 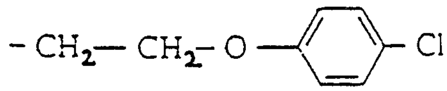

$-CH_2-CH_2-CH_2-SCH_3$. $-CH_2-CH_2-CN$. 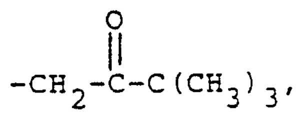,

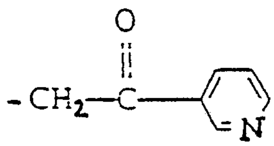 . 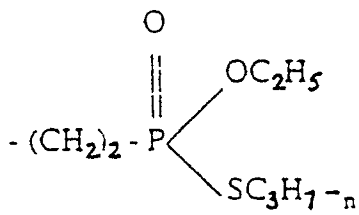 . $-CH_2-Si(CH_3)_3$.

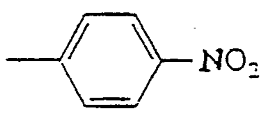 . 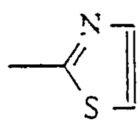 . 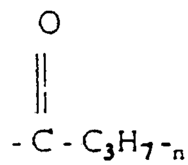 . 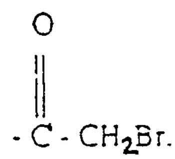 .

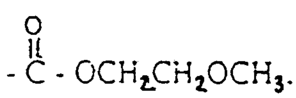 .

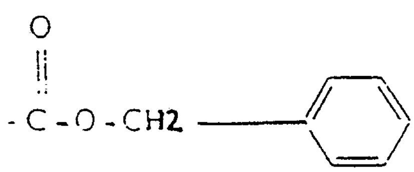 .

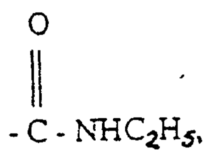 , 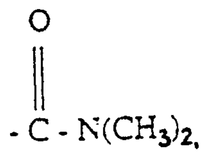 , $-SO_2CH_3$ ,

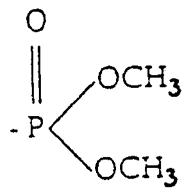 , 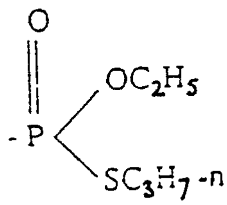 ,

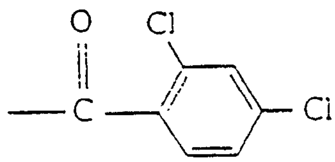 .

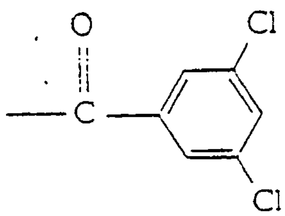 ,

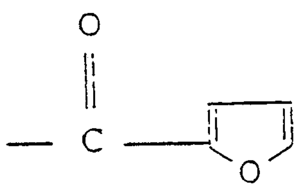 .